# EUROPÄISCHE PATENTSCHRIFT

(11) **EP 2 261 189 B1**
(45) Veröffentlichungstag und Bekanntmachung des Hinweises auf die Patenterteilung: **23.11.2016**
(21) Anmeldenummer: 10183620.3
(22) Anmeldetag: 15.12.2003
(51) Int. Cl.: C04B 35/19, C04B 35/488, A61K 6/06

(54) **MIT NANOSKALIGEM METALLOXID-PULVER VERSETZTE LEUCIT-GLASKERAMIK**
LEUCITE BASED GLASS CERAMIC CONTAINING NANOSCALE METALLIC OXIDE POWDER
VITROCÉRAMIQUE À BASE DE LEUCITE CONTENANT UNE POUDRE NANOMÉTRIQUE D'OXYDE MÉTALLIQUE

(30) Priorität: 30.12.2002 DE 10261717
(43) Veröffentlichungstag der Anmeldung: 15.12.2010
(62) Teilanmeldung aus: 03028805.4
(73) Patentinhaber: Chemichl AG, 9490 Vaduz (LI); Meyer, Gerhard, Prof., Dr., 46483 Wesel (DE)
(72) Erfinder: Meyer, Gerhard, 46483 Wesel (DE); Conrad, Thomas, 44287 Dortmund (DE)
(74) Vertreter: Jostarndt Patentanwalts-AG

(56) Entgegenhaltungen:
- WO-A-00/10509
- WO-A2-99/45888
- DE-A1- 10 049 971
- US-A- 4 798 536
- US-A- 4 978 640
- US-A- 6 155 830

## Beschreibung

Die vorliegende Erfindung betrifft eine Leucit-Glaskeramik, ein zu ihrer Herstellung geeignetes Leucit-Glaskeramik-Pulver, Verfahren zur Herstellung der Leucit-Glaskeramik, ihre Verwendung als Dentalmaterial, ein sie unfassendes Dentalprodukt und die Verwendung von nanoskaligen Metalloxid-Pulvern zur Herstellung der Glaskeramik bzw. des Leucit-Glaskeramik-Pulvers.

Leucit-Glaskeramiken finden ihren Einsatz insbesondere in der Dentaltechnik zur Herstellung von Dentalprodukten, wie beispielsweise Zahnersatz, Zahnstiften oder Zahnwurzelaufbauten. Häufig werden diese Dentalprodukte mittels Presstechnik hergestellt. Dabei wird zunächst ein (Leucit-)Glaskeramik-Pulver in einem Ofen erschmolzen und unter Druck zu Zylindern, die üblicherweise 2 g schwer sind, geformt. Diese Zylinder werden dann in einem speziellen Pressofen in eine Form gepresst, die vom wachsartigen Positiv-Modell gebildet wird, welches vom Zahntechniker angefertigt wurde. Somit wird nach dem Prinzip des so genannten Lost-Wax-Verfahrens eine exakte Kopie des ursprünglichen Wachsmodells aus Glaskeramik hergestellt.

Ein Glaskeramikpulver, das in diesem Verfahren Verwendung finden soll, muss demnach zum einen ausreichend pressbar und zum anderen ausreichend viskos sein, um den durch das Wachsmodell vorgegebenen Raum vollständig auszufüllen. Zugleich ist es wünschenswert, dass die eingesetzten Glaskeramiken über eine hohe Biegefestigkeit verfügen, damit die aus ihnen erhaltenen Dentalprodukte insbesondere der mechanischen Beanspruchung standhalten und nicht ohne Weiteres durchbrechen bzw. Risse bilden.

Leucit-Glaskeramiken sind für die Verarbeitung im Pressofen ausreichend pressfähig und viskos. Es ist jedoch wünschenswert, ihre Biegefestigkeit und Risszähigkeit weiter zu verbessern, um Dentalprodukte mit besseren mechanischen Eigenschaften zur Verfügung stellen zu können.

Es ist daher eine Aufgabe der vorliegenden Erfindung, eine Leucit-Glaskeramik zur Verfügung zu stellen, deren Biegefestigkeit und Risszähigkeit gegenüber den bekannten Leucit-Glaskeramiken verbessert ist. Ferner soll ein Leucit-Glaskeramik-Pulver bereitgestellt werden, aus dem sich eine Leucit-Glaskeramik mit den genannten gewünschten Eigenschaften herstellen lässt.

Diese Aufgabe wird gelöst durch die Bereitstellung eines Leucit-Glaskeramik-Pulvers, das (a) ein Leucit-Glaskeramik-Pulver und (b) ein nanoskaliges Metalloxid-Pulver mit einem d₅₀-Wert von 1 nm bis 200 nm umfasst, wobei das nanoskalige Metalloxid (b) Li₂O, TiO₂, ZnO oder La₂O₃ ist und das das nanoskalige Metalloxid (b) in einer Menge von 30 bis 80 Gew.-% bezogen auf das Gesamtgewicht der Leucit-Glaskeramik vorliegt. Dieses Leucit-Glaskeramik-Pulver erlaubt die Herstellung einer erfindungsgemäßen Leucit-Glaskeramik, umfassend (a) eine Leucit-Glaskeramik; und (b) ein nanoskaliges Metalloxid mit einem d₅₀-Wert von 1 nm bis 200 nm. Eine solche Leucit-Glaskeramik weist im Vergleich zu Leucit-Glaskeramiken ohne Zugabe an nanoskaligem Metalloxid-Pulver eine deutlich höhere Biegefestigkeit unter Erhalt der guten Pressfähigkeit und Viskosität auf. Wird zur Herstellung der Leucit-Glaskeramik statt dem nanoskaligen Metalloxid-Pulver mit der angegebenen Korngröße handelsübliches Metalloxid-Pulver mit Korngrößen im Mikrometer-Bereich verwendet, liegt die Biegefestigkeit dieser Leucit-Glaskeramik teilweise unter der der Leucit-Glaskeramik ohne Zugabe an nanoskaligem Metalloxid-Pulver.

Unter einer Leucit-Glaskeramik ist im Zusammenhang mit der vorliegenden Erfindung insbesondere eine Glaskeramik zu verstehen, die im Wesentlichen aus dem Gerüstsilicat Leucit (K[AlSi₂O₆]) gebildet wird, wobei das Leucit weitere Elemente bzw. Elementoxide, wie z.B. Na, Ca, Ba, Ce, B oder Ti bzw. deren Oxide, umfassen kann.

Leucit-Glaskeramik-Pulver werden z.B. gemäß dem aus der internationalen Patentanmeldung WO 0010509 offenbarten Verfahren hergestellt, sie können jedoch auch nach anderen Verfahren aus Leucit bildenden Ausgangsmaterialien (wie z.B. Oxiden) hergestellt werden.

Die zu schützende Zusammensetzung der Leucit-Glaskeramik (a) der erfindungsgemäßen Leucit-Glaskeramik weist beispielsweise folgende Zusammensetzung auf:

| | |
|---|---|
| SiO₂ | 63-71 Gew.-% (bezogen auf das Gesamtgewicht der Leucit-Glaskeramik (a)); |
| Al₂O₃ | 10-15 Gew.-%; |
| K₂O | 8-10 Gew.-%; |
| Na₂O | 3-8 Gew.-%; |
| CaO | 1-3 Gew.-%; |
| BaO | 0,2-2 Gew.-%; |
| CeO₂ | 0,5-2 Gew.-%; |
| B₂O₃ | 0,5-2 Gew.-%; |
| TiO₂ | 0-1 Gew.-%. |

Der Begriff "nanoskaliges Metalloxid-Pulver" bezeichnet solche Metalloxid-Pulver, deren durchschnittliche Korngröße im Nanometer-Bereich liegt. Die Korngröße wird dabei üblicherweise mittels Laserbeugung bestimmt.

Die Verwendung von ZrO₂-haltigen Metalloxiden als Dotierung von Glaskeramiken ist im Stand der Technik bereits bekannt. So offenbart die EP 690 031 Glaskeramiken, die mindestens zwei Kristallphasen aufweisen, unter anderem ZrO₂ und P₂O₅ umfassen. Es handelt sich hierbei jedoch nicht um Leucit-Glaskeramiken, und die ZrO₂ - Dotierung erfolgt direkt in die Glasschmelze.

In einer bevorzugten Ausführungsform weist die Leucit-Glaskeramik (a) der erfindungsgemäßen Leucit-Glaskeramik folgende Zusammensetzung auf:

| | |
|---|---|
| SiO₂ | 70 Gew.-% (bezogen auf das Gesamtgewicht der Leucit-Glaskeramik (a)); |
| Al₂O₃ | 10 Gew.-%; |
| K₂O | 10 Gew.-%; |
| Na₂O | 5 Gew.-%; |
| CaO | 2 Gew.-%; |
| BaO | 1 Gew.-%; |
| CeO₂ | 1 Gew.-%; |

| | |
|---|---|
| B₂O₃ und TiO₂ | 1 Gew.-%; |

Hierbei handelt es sich um eine handelsübliche Leucit-Glaskeramik K 13 S. Aus einem entsprechenden Glaskeramik-Pulver (a) und einem nanoskaligen Metalloxid-Pulver (b) ist die erfindungsgemäße Leucit-Glaskeramik nach üblichen Verfahren erhältlich.

Es ist weiterhin bevorzugt, wenn in der erfindungsgemäßen Leucit-Glaskeramik das nanoskalige Metalloxid (b) in einer Menge von 30 bis 70 Gew.-% (bezogen auf das Gesamtgewicht der Leucit-Glaskeramik), besonders bevorzugt von etwa 60 Gew.-% vorliegt. Diese Leucit-Glaskeramiken sind beispielsweise durch herkömmliche Verfahren, ausgehend von den entsprechenden Mengen Leucit-Glaskeramik-Pulver (a) und nanoskaligen Metalloxid-Pulver (b) zugänglich. So können sie durch Vermahlen der Pulver in einem Trocken- oder Nassmahl-Verfahren und anschließendes Erschmelzen bei Temperaturen von z.B. 1100 bis 1200 °C erhalten werden. Dabei werden die vermahlenden Pulver auf die gewünschte Sintertemperatur erhitzt und dort für einen festgesetzten Zeitraum, üblicherweise für etwa 5 bis etwa 30 Minuten, gehalten und anschließend abgekühlt. Die auf diese Weise erhaltene Keramik kann dann vom Zahntechniker - wie eingangs dargelegt bzw. in der EP 231 773 A1 erläutert - zum gewünschten Dentalprodukt weiterverarbeitet werden.

Es hat sich außerdem gezeigt, dass die Korngröße des nanoskaligen Metalloxid-Pulvers (b), aus dem zusammen mit dem Leucit-Glaskeramik-Pulver (a) durch übliche Verfahren die erfindungsgemäße Leucit-Glaskeramik erhalten werden kann, bevorzugt zwischen 10 und 100 nm, insbesondere zwischen 20 und 70 nm und besonders bevorzugt etwa 30 bis 60 nm beträgt.

Als nanoskaliges Metalloxid-Pulver ist Li₂O, TiO₂ ZnO oder La₂O₃ geeignet, und zwar besonders bevorzugt mit einer Korngröße von 30 bis 60 nm. Die zur Herstellung der erfindungsgemäßen Leucit-Glaskeramiken verwendeten nanoskaligen Metalloxid-Pulver sind nach allgemein üblichen Syntheseverfahren zugänglich. So können Metalloxid-Pulver z.B. auf verschiedenen chemischen Wegen mittels Sol-Gel-Synthese hergestellt werden. Ein Verfahren ist die Mikroemulsionstechnik nach G. Rinn und H. Schmidt in Ceramic Powder Processing Science (Proceedings of the second International Conference 12-14.10.1988). Weitere Möglichkeiten bieten Y.T. Moon, D.K. Kim, C.H. Kim in J. Am. Ceram. Soc., 78 [4] 1103-106, J.D. Mackenzie in Ultrastructure Processing of Ceramics, Glasses and Composites, 1984, S. 15-26, E.A. Barringer und H.K.Bowen in J. Am. Ceram. Soc. 1982, S.199-201, E. Matijevic in Acc. Chem. Res., 1981, S.22-29, Fegley und Barringer in Mat. Res. Soc. Proc. , 1984, S. 187-197. Alternativ kann die Metallsalzsole durch Flammpyrolyse gemäß S. Begand und S. Ambrosius in DKG, S. D12-D16, 1998 und in Chemie Ingenieur Technik, S. 746-749; 1998 nanoskalige Metalloxidpulver liefern. Letztlich können die nanoskaligen Metalloxid-Pulver auch nach einem Plasmasynthese-Verfahren gemäß DE 3339490 A1 erzeugt werden.

Als besonders vorteilhaft im Hinblick auf die gewünschte Steigerung der Biegefestigkeit hat sich erwiesen, die erfindungsgemäßen Leucit-Glaskeramiken nach ihrer Herstellung, z.B. im Pressofen, und der Sinterung einer chemischen Härtung zu unterziehen. Dabei wird die Keramik für einen gewissen Zeitraum, z.B. 1 bis 24 h, insbesondere 4 bis 12 h, bevorzugt etwa 8 h, bei erhöhter Temperatur, z.B. 200 bis 800 °C, insbesondere 300 bis 600 °C, bevorzugt 420 bis 550 °C, einer Lösung eines Härtungsmittels ausgesetzt. Dabei handelt es sich bevorzugt um eine (wässrige) Lösung eines härtenden Salzes, das vorzugsweise aus der Gruppe ausgewählt ist, die NaCl, NaNO₃, KCl und KNO₃ umfasst. Durch diese Härtung können die ohnehin bereits erheblich gesteigerten Biegefestigkeiten der erfindungsgemäßen Leucit-Glaskeramiken nochmals erhöht, teilweise sogar verdoppelt werden.

Die erfindungsgemäßen Leucit-Glaskeramiken sind aufgrund ihrer hohen Biegefestigkeit und Risszähigkeit hervorragend als Dentalwerkstoff geeignet. Ferner zeigen sie eine gute Temperaturwechselbeständigkeit. Sie sind außerdem bei Temperaturen unterhalb von 1200 °C verarbeitbar, wozu insbesondere das für die Herstellung von Dentalprodukten vorteilhafte Heißpressverfahren (Lost-Wax-Verfahren) im viskosen Zustand eingesetzt wird, wie es z.B. aus der EP 231 773 A1 bekannt ist. Eine Formung von herkömmlichen hochfesten Glaskeramiken ist bei diesen niedrigen Temperaturen häufig nicht möglich. Auch erweist sich als Vorteil, dass die erfindungsgemäßen Leucit-Glaskeramiken - im Gegensatz zu vielen herkömmlichen Glaskeramiken - nicht mit der Einbettmasse reagieren, die bei der Herstellung von geformten Dentalprodukten mittels Heißpressverfahren eingesetzt wird. Dies ist von großem Vorteil für den sie verarbeitenden Dentaltechniker. Schließlich haften die erfindungsgemäßen Leucit-Glaskeramiken sehr gut an hochfesten reinen ZrO₂-Keramiken, was besonders für die Verwendung in der Dentaltechnik wichtig ist. Es ist z.B. möglich, an einem hochfesten ZrO₂-Keramik-Wurzelstift direkt nach individueller Formgebung - also in Abhängigkeit von der jeweiligen Kavität, eine passfähige Leucit-Glaskeramik anzupressen. So wird der ZrO₂-Wurzelstift fest am Zahn verankert, und ein weiterer Zahnaufbau kann vorgenommen werden.

Aus den genannten Eigenschaften ergibt sich, dass die erfindungsgemäße Leucit-Glaskeramik auch als Dentalmaterial oder daraus geformtes Dentalprodukt oder als Bestandteil von Dentalmaterial oder von daraus geformtem Dentalprodukt eingesetzt wird. Bevorzugte Dentalprodukte sind dabei Zahnwurzelkonstruktionen wie Zahnwurzelaufbauten oder Zahnwurzelstifte.

Nachfolgend wird die Erfindung anhand von Beispielen näher erläutert, ohne dass der Schutzumfang der Erfindung durch sie beschränkt werden soll.

### Vergleichsbeispiele

### Vorbemerkung

Die verwendeten Materialien sind kommerziell erhältlich bzw. durch bekannte Herstellungsverfahren herstellbar.

Korngrößen wurden mittels TEM bestimmt; die Bestimmung der spezifischen Oberfläche erfolgte nach BET, die Bestimmung der Kristallphasen durch Röntgendiffraktometrie.

### Beispiel 1:

### (Vergleichsbeispiel)

### Herstellung von nanoskaligen ZrO₂-Pulvern nach dem Plasmasynthese-Verfahren

1757g ZrCl₄ und 190g YCl₃ • 6 H₂O werden zu 1 kg nanoskaliges ZrO₂, stabilisiert mit 3 mol-% Y₂O₃, umgesetzt, wobei dieses eine durchschnittliche Korngröße von 50 nm und einer spezifischen Oberfläche von 26 ± 2 m²/g auf.

Charakteristische Eigenschaften dieses ZrO₂-Pulvers sind in Tabelle 1 wiedergegeben.

**Tabelle 1**

| **Nr.** | **Stabilisator** | **Durchschnittliche Korngröße [nm]** | **Spezifische Oberfläche [m²/g]** | **Kristallphasen ZrO₂** |
|---|---|---|---|---|
| 1 | - | 30 | 35±5 | 25 % monoklin |
| | | | | 75 % tetragonal |
| 2 | 9 mol-% MgO | 55 | 20 ±2 | 10 % monoklin |
| | | | | 90 % tetragonal |
| 3 | 3 mol-% Y₂O₃ | 50 | 26 ±2 | 10 % monoklin |
| | | | | 90 % tetragonal |

### Beispiel 2:

### (Vergleichsbeispiel) Herstellung von Leucit-Glaskeramiken

Das Leucit-Glaskeramik-Pulver K 13 S und ein nanoskaliges ZrO₂-Pulver aus Beispiel 1 wurden 24h lang in Ethanol gemahlen und gemischt (Pulvermenge: 100g) Zur Ermittlung der 3-Punkt-Biegefestigkeit nach der Dentalkeramik-Norm EN ISO 6872 wurden aus dem Pulvergemisch im Dentalpressofen Lectra Press der Fa. UGIN'dentaire Biegebruchstäbchen (Abmessungen 30 x 4 x 1,3 mm) angefertigt. Als Vergleichsprobe wurde analog ein Biegebruchstäbchen aus einem Leucit-Glaskeramik-Pulver K 13 S ohne nanoskaliges Metalloxidpulver hergestellt [Aufheizrate: 60 °C/min, Sinterungsendtemperatur: 760°C und Haltezeit bei Sinterungsendtemperatur: 5 min]
welches unter zur Hilfenahme einer Weibull-Statistik Biegefestigkeit von 107 MPa ergab. Alle Proben wiesen eine unpolierte Oberfläche auf. Die getesteten Proben und ihre Biegefestigkeiten sind in Tabelle 2 wiedergegeben.

**Tabelle 2**

| **Probe** | **Nanoskaliges ZrO₂ nach Beispiel 1** | **ZrO₂-Gehalt [Gew.-%]** | **Aufheizrate [°C/min]** | **Sinterungsendtemperatur [° C]** | **Haltezeit bei Sinterungsendtemperatur [°C]** | **Biegefestigkeit [MPa]** |
|---|---|---|---|---|---|---|
| A | 1 | 60 | 90 | 1150 | 5 | 214 |
| B | 1 | 60 | 90 | 1150 | 7 | 198 |
| C | 1 | 60 | 90 | 1150 | 10 | 177 |
| D | 2 | 80 | 140 | 1150 | 10 | 152 |
| E | 2 | 80 | 140 | 1150 | 14 | 161 |
| F | 3 | 60 | 90 | 1150 | 5 | 130 |
| G | 3 | 60 | 90 | 1150 | 7 | 132 |

Anhand der Messwerte für die Biegefestigkeit in Tabelle 2 ist der festigkeitssteigernde Einfluss der Zugabe mit den erfindungsgemäßen Nanomaterialien eindeutig nachweisbar.

Überraschenderweise zeigte sich, dass auch mit einer entsprechend hohen Zugabe, die ja einem Wechsel im Matrix-Dotiermittel-Verständnis entspricht, eine Verpressbarkeit der Materialmischung gewährleistet bleibt.

### Beispiel 3

### (Vergleichsbeispiel)

### Härtung einer Leucit-Glaskeramik nach Beispiel 2

Eine Leucit-Glaskeramik nach Beispiel 2 (Probe A) wurde für einer chemischen Härtung mit KNO₃ unterzogen [Aufheizrate: 5°C/min, Haltezeit: 8 h bei 480 °C Abkühlrate: 3 °C/min). Die so erhaltene gehärtete Leucit-Glaskeramik wies eine Biegefestigkeit von 437 MPa auf.

## Patentansprüche

1. Leucit-Glaskeramik-Pulver, umfassend
(a) ein Leucit-Glaskeramik-Pulver; und
(b) ein nanoskaliges Metalloxid-Pulver mit einem d₅₀-Wert von 1 nm bis 200 nm, wobei dass das nanoskalige Metalloxid (b) Li₂O, TiO₂, ZnO oder La₂O₃ ist und das nanoskalige Metalloxid (b) in einer Menge von 30 bis 80 Gew.-% bezogen auf das Gesamtgewicht der Leucit-Glaskeramik vorliegt.

2. Leucit-Glaskeramik, umfassend
(a) eine Leucit-Glaskeramik; und
(b) ein zugegebenes nanoskaliges Metalloxid mit einem d₅₀-Wert von 1 nm bis 200 nm, wobei das nanoskalige Metalloxid (b) Li₂O, TiO₂, ZnO oder La₂O₃ ist und das nanoskalige Metalloxid (b) in einer Menge von 30 bis 80 Gew.-% bezogen auf das Gesamtgewicht der Leucit-Glaskeramik vorliegt.

3. Leucit-Glaskeramik nach Anspruch 2, **dadurch gekenn-zeichnet**, dass die Leucit-Glaskeramik folgende Zusammensetzung aufweist:
| | |
|---|---|
| SiO₂ | 70 Gew.-% (bezogen auf das Gesamtgewicht der Leucit-Glaskeramik (a)); |
| Al₂O₃ | 10 Gew.-%; |
| K₂O | 10 Gew.-%; |
| Na₂O | 5 Gew.-%; |
| CaO | 2 Gew.-%; |
| BaO | 1 Gew.-%; |
| CeO₂ | 1 Gew.-%; |
| B₂O₃ und TiO₂ | 1 Gew.-%. |

4. Leucit-Glaskeramik nach einem der Ansprüche 2 bis 3, **dadurch geken nzeichnet,** dass die Korngröße des nanoskaligen Metalloxids (b) 10 bis 200 nm, insbesondere 20 bis 100 nm, besonders bevorzugt 30 bis 60 nm beträgt.

5. Leucit-Glaskeramik nach einem der Ansprüche 2 bis 4, **da-durch gekennzeichnet,** dass das nanoskalige Metalloxid-Pulver (b) nach einem Plasmasynthese-Verfahren hergestellt worden ist und eine überdurchschnittlichen Anteil kleinster Nanoteilchen < 60 nm und dementsprechend eine große aktive Oberfläche hat.

6. Leucit-Glaskeramik nach einem der Ansprüche 2 bis 5, **dadurch geken nzeichnet**, dass sie nach der Herstellung einer chemischen Härtung unterzogen worden ist.

7. Verfahren zur Herstellung der Leucit-Glaskeramik nach einem der Ansprüche 2 bis 6, **dadurch gekennzeichnet, dass** das Leucit-Glaskeramik-Pulver nach Anspruch 1 einer Sinterung unterzogen wird.

8. Verfahren nach Anspruch 7, **dadurch gekennzeichnet, dass die** Leucit-Glaskeramik nach der Sinterung einer chemischen Härtung unterzogen wird.

9. Verwendung der Leucit-Glaskeramik nach einem der Ansprüche 2 bis 6, **dad urch gekennzeichnet,** dass es als Dentalmaterial oder daraus geformtes Dentalprodukt oder als Bestandteil von Dentalmaterial oder von daraus geformtem Dentalprodukt verwendet wird.

## Claims

1. A leucite glass ceramic powder, comprising
(a) a leucite glass ceramic powder; and
(b) a nanoscale metal oxide powder with a d₅₀ value of 1 nm to 200 nm, whereby the nanoscale metal oxide (b) is Li₂O, TiO₂, ZnO or La₂O₃, and the nanoscale metal oxide (b) is present in a quantity of 30% to 80% by weight relative to the total weight of the leucite glass ceramic.

2. A leucite glass ceramic, comprising
(a) a leucite glass ceramic powder; and
(b) an added nanoscale metal oxide powder with a d₅₀ value of 1 nm to 200 nm, whereby the nanoscale metal oxide (b) is Li₂O, TiO₂, ZnO or La₂O₃, and the nanoscale metal oxide (b) is present in a quantity of 30% to 80% by weight relative to the total weight of the leucite glass ceramic.

3. The leucite glass ceramic according to claim 2,
**characterized in that**
the leucite glass ceramic has the following composition:
| | |
|---|---|
| SiO₂ | 70% by weight (relative to the total weight of the leucite glass ceramic (a)); |
| Al₂O₃ | 10% by weight; |
| K₂O | 10% by weight; |
| Na₂O | 5% by weight; |
| CaO | 2% by weight; |
| BaO | 1% by weight; |
| CeO₂ | 1 % by weight; |
| B₂O₃ and TiO₂ | 1 % by weight. |

4. The leucite glass ceramic according to claim 2 or 3,
**characterized in that**
the particle size of the nanoscale metal oxide (b) is 10 nm to 200 nm, especially 20 nm to 100 nm, especially preferable 30 nm to 60 nm.

5. The leucite glass ceramic according to one of claims 2 to 4,
**characterized in that**
the nanoscale metal oxide powder (b) has been produced by means of a plasma synthesis method, and it has an above-average fraction of minute nanoparticles < 60 nm, thus correspondingly having a large active surface area.

6. The leucite glass ceramic according to one of claims 2 to 5,
**characterized in that**,
after the production process, it undergoes chemical hardening.

7. A method for the production of the leucite glass ceramic according to one of claims 2 to 6,
**characterized in that**
the leucite glass ceramic powder according to claim 1 undergoes a sintering process.

8. The method according to claim 7,
**characterized in that**,
after the sintering process, the leucite glass ceramic undergoes chemical hardening.

9. The use of the leucite glass ceramic according to one of claims 2 to 6,
**characterized in that**
it is used as a dental material or as a dental product made thereof, or as a component of dental material or of a dental product made thereof.

## Revendications

1. Poudre de vitrocéramique de leucite, comprenant :
(a) une poudre de vitrocéramique à base de leucite et
(b) une poudre d'oxyde métallique de taille nanométrique ayant une valeur d₅₀ de 1 nm à 200 nm, l'oxyde métallique de taille nanométrique (b) étant du Li₂O, du TiO₂, du ZnO ou du La₂O₃ et l'oxyde métallique de taille nanométrique (b) étant présent en quantité de 30 à 80 % en poids rapportés au poids total de la vitrocéramique de leucite.

2. Vitrocéramique à base de leucite, comprenant :
(a) une vitrocéramique à base de leucite et
(b) un oxyde métallique de taille nanométrique ajouté avec une valeur d₅₀ de 1 nm à 200 nm, l'oxyde métallique nanométrique (b) étant du Li₂O, du TiO₂, du ZnO ou du La₂O₃ et l'oxyde métallique nanométrique (b) étant présent en quantité de 30 à 80 % en poids rapportée au poids total de la vitrocéramique de leucite.

3. Vitrocéramique à base de leucite selon la revendication 2, **caractérisée en ce que** la vitrocéramique à base de leucite présente la composition suivante :
| | |
|---|---|
| SiO₂ | 70 % en poids (rapportés au poids total de la vitrocéramique de leucite (a)) ; |
| Al₂O₃ | 10 % en poids; |
| K₂O | 10% en poids; |
| Na₂O | 5 % en poids; |
| CaO | 2 % en poids; |
| BaO | 1 % en poids; |
| CeO₂ | 1 % en poids; |
| B₂O₃ et TiO₂ | 1 % en poids; |

4. Vitrocéramique de leucite selon l'une des revendications 2 à 3, **caractérisée en ce que** la granularité de l'oxyde métallique nanométrique (b) est de 10 à 200 nm, en particulier de 20 à 100 nm, particulièrement préférentiellement de 30 à 60 nm.

5. Vitrocéramique de leucite selon l'une des revendications 2 à 4, **caractérisée en ce que** la poudre d'oxyde métallique de taille nanométrique (b) a été fabriquée selon un procédé de synthèse plasma et présente une fraction supérieure à la moyenne de nanoparticules infimes < 60 nm et une surface active d'une taille en conséquence.

6. Vitrocéramique de leucite selon l'une des revendications 2 à 5, **caractérisée en ce qu'**elle subit un durcissement chimique après la fabrication.

7. Procédé de fabrication de la vitrocéramique de leucite selon l'une des revendications 2 à 6, **caractérisé en ce que** la poudre de vitrocéramique de leucite selon la revendication 1 subit un frittage.

8. Procédé selon la revendication 7, **caractérisé en ce que** la vitrocéramique de leucite subit un durcissement chimique après le frittage.

9. Utilisation de la vitrocéramique de leucite selon l'une des revendications 2 à 6, **caractérisée en ce qu'**elle est utilisée en tant que matériau dentaire ou produit dentaire formé sur cette base ou composant de matériau dentaire ou de produit dentaire formé sur cette base.
